# EUROPEAN PATENT APPLICATION

(11) **EP 4 563 723 A1**
(43) Date of publication of application: **04.06.2025**
(21) Application number: 23845737.8
(22) Date of filing: 31.07.2023
(51) Int. Cl.: C23F 3/00, A61F 2/82

(54) **MEDICAL DEVICE POLISHING MEMBER AND MANUFACTURING METHOD THEREFOR**

(30) Priority: 29.07.2022 CN 202210908081
(71) Applicant: Biotyx Medical (Shenzhen) Co., Ltd., Shenzhen, Guangdong 518000 (CN)
(72) Inventor: LIU, Ziqiang, Shenzhen, Guangdong 518000 (CN); ZHANG, Deyuan, Shenzhen, Guangdong 518000 (CN); ZHANG, Gui, Shenzhen, Guangdong 518000 (CN)
(74) Representative: Prinz & Partner mbB
(86) International application number: PCT/CN2023/110244
(87) International publication number: WO 2024/022533

(57) **Abstract**

Provided are a medical device polishing member and a preparation method thereof. The medical device polishing member has a mass loss rate of 20-60% in the polishing process, and has good mechanical properties, surface properties, and biosafety. Further provided is a preparation method of the medical device polishing member, and the preparation method has the advantages such as high polishing precision, simple and convenient operation, and low costs.

## Description

### Technical Field

The present invention relates to the field of chemical polishing, and particularly to a medical device polishing member and a polishing method thereof.

### Background Art

Currently, polishing methods commonly used in the field of medical devices include an electrochemical polishing method, an abrasive polishing method, and a chemical polishing method. For example, in patent CN102356184B, a method and solution for electropolishing stents made of high-strength medical alloys are disclosed. In this method, one or more conductive adapters need to be attached to each stent, and meanwhile, cathode and anode current conducting members are connected, which not only has high requirements for the device, but also discloses in the embodiments thereof that the whole polishing process needs to be repeated four times. In addition, after polishing, a new polished surface formed after polishing is performed passivation treatment by immersing the rinsed stent into a passivation solution, thereby preventing the new polished surface from being rapidly oxidized in the air. The process disclosed in this patent is complicated, and the operation is cumbersome. In patent application US5746691A, an abrasive polishing method is disclosed. Polishing is achieved by contacting each surface of a stent with a fluid abrasive. However, the abrasive tends to leave residues and easily causes scratches on the surface of the polishing member, resulting in that the surface of the polishing member is not smooth enough. In patent application US4704126A, a method of chemically polishing a medical implant made of titanium or a titanium alloy is disclosed. In this method, a polishing solution formed by lactic acid or glycerol, hydrofluoric acid, and nitric acid and the implant move relative to one another to achieve the purpose of polishing the medical implant made of titanium or a titanium alloy. However, in this polishing method, the polished surface is not bright enough, the polishing time is short, it is not easy to control the quality and size precision, and the size stability and reproducibility of the polished product are not high. The finished products after polishing under the same conditions have a great error in terms of size and quality, which is not suitable for large-scale industrial production. In addition, the polishing method is suitable for the polishing of titanium or the titanium alloy, but is not necessarily suitable for the polishing of other metals or metal alloys.

In addition, the related art disclosed so far mainly discusses how to remove the oxidation layer on the surface of the medical device polishing member and ensure that the surface of the polishing member is smooth, but it does not address how to improve the mechanical properties of the medical device through the polishing process, thereby improving the product yield and reducing potential safety hazards.

### Summary of the Invention

In order to overcome the above-mentioned drawbacks existing in the related art, the present invention provides a medical device polishing member and a preparation method thereof. The medical device polishing member has a smooth surface without burr, round edges, good mechanical properties, relatively high safety during implantation into the human body, small damage to the human body, and high polishing precision.

According to an aspect of the present invention, there is provided a medical device polishing member with good mechanical properties by breaking the limitation of internal defects of a material. The medical device polishing member has a mass loss rate of 20-60% in the polishing process. Further, the medical device polishing member has a mass loss rate of 20-55%, 20-45%, 20-50%, 15-50%, 25-55%, 25-50%, 25-60%, 30-60%, 40-60%, 35-60%, 42-60%, or 40.1-60% in the polishing process.

The "polishing member" described in the present invention refers to a finished medical device product after polishing.

The "mass loss rate of the medical device polishing member in the polishing process" described in the present invention refers to a ratio of the decrease in the mass of the medical device polishing member after polishing to the mass of the medical device polishing member before polishing, i.e., the mass loss rate of the medical device polishing member in the polishing process = (the mass of the medical device polishing member before polishing - the mass of the medical device polishing member after polishing)/the mass of the medical device polishing member before polishing.

Meanwhile, the meaning of the mass loss rate mentioned in the present invention is equivalent to the meaning of a polishing removal rate, and "polishing removal amount" refers to the amount of mass loss of the medical device polishing member after polishing, i.e., the polishing removal amount of the medical device polishing member in the polishing process = the mass of the medical device polishing member before polishing - the mass of the medical device polishing member after polishing.

In the above-mentioned polishing member provided by the present invention, a single-side removal depth after polishing an inner wall, outer wall, and side wall of the polishing member is ≥ 1.5 µm. Furthermore, the single-side removal depth after polishing the inner wall, outer wall, and side wall of the polishing member is 2.5 µm, 3 µm, 3.5 µm, 4 µm, 4.5 µm, 5 µm, 5.5 µm, 6 µm, 6.5 µm, 7 µm, 7.5 µm, 8 µm, 8.5 µm, 9 µm, 9.5 µm, 10 µm, or more. Further, the single-side removal depth after polishing the inner wall, outer wall, and side wall of the polishing member is controlled within 10-30 µm or even more than 30 µm.

In the present invention, according to the internal structure of each polishing member, the total polishing removal amount of the polishing member and the polishing removal amounts of all surfaces (inner wall, outer wall, and side wall) of the polishing member are sufficiently controlled to ensure that the structural defects or cracks existing inside the material may be sufficiently polished and removed, thus greatly improving the mechanical properties of the medical device polishing member, and preventing a series of safety problems caused by the failure in mechanical properties of the finally prepared medical device due to the internal defects of the polishing member.

The single-side removal depth in the present invention refers to a half of the total width or thickness reduction in an axial or longitudinal direction of the medical device. For example, when the medical device is a stent, the single-side removal depth is a half of the width or thickness reduction of a stent rod.

In the present invention, according to the principle of the short board of the water bucket, the thicknesses and other parameters of various parts of the medical device polishing member shall be consistent as much as possible by controlling the polishing uniformity of various parts of the medical device polishing member to prevent the short plate from being generated due to the mechanical property of a certain part of the medical device polishing member greatly reduced caused by the overpolishing of a part of the medical device polishing member, thereby preventing the reduction of the overall mechanical property of the medical device polishing member, and avoiding the problem that the quality of the medical device polishing member is not up to standard during use. Therefore, in the present invention, the polishing removal amount of various parts of the medical device polishing member in the polishing process is sufficiently controlled to ensure that the polishing uniformity of the polishing member may reach 85% or more. Preferably, the polishing uniformity of the polishing member is controlled to 90%, 95%, even 98% or more, thereby ensuring that the entire medical device polishing member has no weak parts and has good mechanical properties.

It should be specifically noted that the polishing uniformity of the polishing member described in the present invention refers to the relatively uniform size of the polished member after polishing. That is, the polishing uniformity refers to a ratio of the polished width/thickness Rᵢ of the polishing member at the same part after polishing to the average of the polished width/thickness of the polishing member at the same part after polishing, i.e., Rᵢ/R_{average}. For example, the polishing uniformity of various parts of the medical device polishing member in the polishing process may reach 85% or more, i.e., Rᵢ/R_{average} ≥ 85%.

It should be noted that "the same part" described in the present invention refers to the part of the same category. For example, in a vascular stent, the stent includes connecting rods, supporting rods, etc. The connecting rods at different positions are regarded as the same position, the supporting rods at different positions are regarded as the same part, and the connecting rods and the supporting rods at different positions are not regarded as the same part.

The present invention not only discusses how to improve the mechanical properties of the medical device polishing member, but also prevents, as far as possible, a series of safety problems, such as thrombus, caused by the rough surface of the medical device polishing member in view of the biosafety of the polishing member. Therefore, the present invention also provides a medical device polishing member with a very smooth and round surface to ensure that the device will not cause additional damage to the human body during implantation, thereby sufficiently improving the safety of the device.

In the above-mentioned medical device polishing member provided by the present invention, the polishing member has a smooth bright surface after being magnified 100 times under an optical electron microscope. Further, the polishing member still has a smooth bright surface after being magnified 200 times or 250 times under the optical electron microscope.

In the above-mentioned polishing member provided by the present invention, the polishing member has a slight scratch on a surface after being magnified 500 times under a scanning electron microscope (SEM), where a ratio of a scratch area to a surface area of the entire polishing member is ≤ 20%. Further, according to the polishing member provided by the present invention, the scratch area on the surface of the polishing member is not more than 15%, 10%, or even 6% of the surface area of the entire polishing member under a magnification of 500 times under the SEM.

In the above-mentioned polishing member provided by the present invention, a scratch depth h of the polishing member is ≤ 2.5 µm. Further, the scratch depth of the polishing member may be less than 1 µm. Further, the scratch depth of the polishing member may be less than 0.5 µm or less than 0.3 µm.

In the above-mentioned polishing member provided by the present invention, the roughness Sa of a surface of the medical device polishing member is ≤ 50 nm. Further, the roughness Sa of the surface of the medical device polishing member is not more than 30 nm. Further, the roughness Sa of the surface of the medical device polishing member is not more than 20 nm.

In the above-mentioned polishing member provided by the present invention, a thin protective film is attached to a polished surface of the polishing member so that the surface of the polishing member has a relatively good stability, is not easily oxidized by air, etc., and the stability of the appearance and property of the surface of the device polishing member during transit and storage is ensured.

In the above-mentioned polishing member provided by the present invention, the protective film attached to the surface of the polishing member may be an inorganic protective film or an organic protective film. That is, in some examples, the protective film attached to the surface of the polishing member is made of an inorganic material, and in other examples, the protective film attached to the surface of the polishing member is made of an organic material. However, whether it is an inorganic or organic protective film, the protective film must have biosafety and will not cause some negative effects on the human body after implantation.

In the above-mentioned polishing member provided by the present invention, each edge of the polishing member is round and smooth. Further, the edges of the medical device polishing member have circular arc shapes and are very round and smooth without any spikes or protrusions.

In the above-mentioned polishing member provided by the present invention, a mass of the polishing member is not more than 0.5 kg. Further, the mass of the polishing member is ≤ 0.25 kg. Further, the mass of the polishing member is ≤ 0.10 kg.

In the above-mentioned polishing member provided by the present invention, a mass-to-volume ratio of the medical device polishing member is 0.001-10 g/cm³. Further, the mass-to-volume ratio of the medical device polishing member is 0.001-5 g/cm³. Further, the mass-to-volume ratio of the medical device polishing member is 0.001-0.4 g/cm³. Further, the mass-to-volume ratio of the medical device polishing member is 0.002-0.3 g/cm³. Further, the mass-to-volume ratio of the medical device polishing member is 0.005-0.25 g/cm³.

In the above-mentioned polishing member provided by the present invention, the polishing member belongs to one of an interventional device or an implant device.

In the above-mentioned polishing member provided by the present invention, the polishing member may be a device having a simple structure, such as a patch, or a device having a very complicated structure, such as a stent.

The medical device of the present invention includes an interventional material/device or an implant material/device for use in vivo.

In the above-mentioned polishing member provided by the present invention, the medical device polishing member is made of one of degradable metals or metal alloys. The medical device includes any one of a vascular stent, a heart valve, a non-vascular endoluminal stent, an occluder, an orthopedic implant, a dental implant, a respiratory implant, a gynecological implant, an andrological implant, a suture, or a bolt. The orthopedic implant includes a bone nail and a bone plate.

The polishing member provided by the present invention is an iron base or iron-based alloy. Further, the polishing member of the present invention is a small-sized and complex-structured product.

In the above-mentioned polishing member provided by the present invention, the polishing member is a degradable medical device, including but not limited to degradable metal or alloy materials, such as degradable materials mainly containing iron, magnesium and zinc, including but not limited to degradable materials containing pure iron, pure magnesium, and pure zinc, and not limited to iron, magnesium, and zinc alloy materials doped with other elements, such as alloys doped with nitrogen, hydrogen, oxygen, sulfur, phosphorus, and other non-metal or metal elements.

In the above-mentioned technical solution provided by the present invention, an iron-containing medical device is made of pure iron or an iron-based alloy with a carbon content of not more than 2.11 wt%.

According to another aspect of the present invention, there is provided a method for improving the mechanical properties of the medical device through a polishing process. The method may sufficiently remove cracks and other defect structures deep inside the polishing member, thereby improving the mechanical properties of the whole polishing member, such as a stent, and preventing the occurrence of fractures during the expansion process. Meanwhile, the method can further improve the uniformity and stability of the mechanical properties of the polishing member by controlling the polishing uniformity of various parts of the polishing member, thereby avoiding excessive polishing removal rate at one part of the polishing member and small polishing removal amounts at other parts. Therefore, the situation where the fracture at the position where the removal amount is too large further deteriorates the overall mechanical properties of the entire polishing member, finally causing quality problems such as fractures of the medical device during use and a series of potential safety hazards, is avoided. That is, this polishing process may sufficiently avoid problems such as poor mechanical properties caused by the defects of the internal structure of the product and the uneven structure of various parts, thereby sufficiently improving the product yield and ensuring the biosafety of the product to reach the standard. Further, this process may ensure that the medical device polishing member has a smooth surface and round edges to reduce the corresponding potential safety hazards after the device is implanted into the human body. In addition, the polishing process is simple in steps, does not require complicated device, and does not require complicated operation process. A polishing member with high mechanical properties, high uniformity, high smoothness, and round edges may be prepared directly by a one-step polishing method, and a thin protective film may be directly formed on the surface of the polishing member to improve the surface stability of the polishing member.

In the present invention, the mechanical properties and surface properties of the medical device polishing member is sufficiently improved by controlling the composition formulation in the polishing process and the polishing process parameters. The present invention specifically provides a polishing method for preparing the above-mentioned medical device polishing member, including the steps of:
a) performing polishing pretreatment on a device base;
b) placing a pretreated device base into a polishing solution containing an acidic polishing composition for polishing; and
c) cleaning a polished device base to obtain a device polishing member.

The polishing method for the medical device polishing member provided by the present invention is not limited to the specific implementation method thereof and may be a pure chemical polishing method, a chemical mechanical polishing method, and an electrochemical polishing method. However, most preferably, the polishing method of the medical device polishing member provided by the present invention is suitable for the pure chemical polishing method.

In the above-mentioned polishing method provided by the present invention, a pH value of the polishing solution in step b) is ≤ 1. Further, a pH value of the polishing composition used in the present invention is not more than 0.8 nor more than 0.5. Further, the pH value of the polishing composition used in the present invention is not more than 0.2.

In the above-mentioned polishing method provided by the present invention, the viscosity of the polishing solution in step b) is 1-4 mPa s. Further, the viscosity of the polishing solution in step b) is 1.2-4 mPa s, or even 1.6-3.8 mPa s.

In the above-mentioned polishing method provided by the present invention, a temperature of the polishing in step b) is 20-35°C.

In the above-mentioned polishing method provided by the present invention, a time of the polishing in step b) is 1-20 min. Further, the time of the polishing in step b) may be 1-10 min, 1-10 min, 5-10 min, 5-20 min, 2-15 min, 2-10 min, 10-20 min, or 12-18 min.

In the above-mentioned polishing method provided by the present invention, a flow rate of the polishing solution in the polishing process in step b) is 0.1-1.5 m/s. Further, the flow rate of the polishing solution in the polishing process in step b) is 0.1-1.0 m/s. Further, the flow rate of the polishing solution in the polishing process in step b) is 0.1-0.8 m/s.

In the above-mentioned polishing method provided by the present invention, a flow manner of the polishing solution in the polishing process in step b) includes a swirling or linear flow.

In the above-mentioned polishing method provided by the present invention, the polishing composition includes an acid, an oxidizing agent, a viscosity agent, and a surface protecting agent; based on a total mass fraction of the polishing composition being 100%, the composition of components is as follows:

| Components | Mass percentage content (.wt%) |
|---|---|
| Acid | 40-70% |
| Oxidizing agent | 5-10% |
| Viscosity agent | 1.5-7% |
| Water | Balance |

In the present invention, specific types of inorganic acids and strong oxidizing agents are adopted, and the contents thereof are controlled so that the oxidation and corrosion rates of the surface of a pre-polishing member may be sufficiently controlled, and meanwhile, the removal amount of the surface of the device may be increased to sufficiently remove cracks and the like generated inside the pre-polishing member due to the preceding process, thereby improving the mechanical properties of the polishing member to meet the requirements of various devices for the mechanical properties of the polishing member. In addition, the polishing efficiency of the polishing member may be sufficiently improved, and the polishing cost may be reduced.

The acid in the polishing composition provided by the present invention is selected from at least one of sulfuric acid, phosphoric acid, nitric acid, perchloric acid, or hydrofluoric acid. The strong inorganic oxidizing agent is selected from at least one of sulfate, perchloric acid and salts thereof, perbromic acid and salts thereof, periodic acid and salts thereof, permanganic acid and salts thereof, peroxy acid salts, iodic acid and salts thereof, chlorous acid and salts thereof, hypochlorous acid and salts thereof, hypoiodous acid and salts thereof, oxybromic acid and salts thereof, percarbonate, bromic acid and salts thereof, chloric acid and salts thereof, hydrogen peroxide, potassium nitrate, or sodium nitrate.

In the above-mentioned technical solution provided by the present invention, the viscosity of the polishing solution is 1.1-4 mPa s. Further, the viscosity of the polishing solution is 1.5-4 mPa s, 1.2-4 mPa s, 1.4-4 mPa s, 1.6-4 mPa s, 1.2-3.5 mPa s, 1.4-3.5 mPa s, 1.6-3.5 mPa s, 1-3.5 mPa s, 1.8-3.5 mPa s, 2.0-3.5 mPa s, 1.2-3.4 mPa s, 1.2-3.4 mPa s, 1.2-3.2 mPa s, 1.2-3.6 mPa s, 1.2-3.0 mPa s, or 1.4-3.3 mPa s. Further, the viscosity of the polishing solution is 1.4-3.0 mPa s, 1.4-3.2 mPa s, 1.6-3.0 mPa s, or 1.6-2.9 mPa s. In some examples of the present invention, the viscosity of the polishing solution is 1.9 mPa s, 1.7 mPa s, 2.1 mPa s, 2.3 mPa s, 2.5 mPa s, 2.7 mPa s, 2.9 mPa s, 3.0 mPa s, 3.1 mPa s, or 3.3 mPa s. In other examples, the viscosity of the polishing solution is 3.5 mPa s, 3.7 mPa s, or 3.9 mPa s.

In the above-mentioned technical solution of the present invention, the viscosity of the polishing solution is further controlled using a viscosity agent. A mass percentage content of the viscosity agent is 1.5%-7%. Further, the mass percentage content of the viscosity agent is 2%-7%, 2.5%-7%, 3%-7%, 3.5%-7%, 4.5%-7%, 4%-7%, 3.5%-6.5%, 2.5%-6.5%, 2.5%-7%, 2%-6.5%. Further, the mass percentage content of the viscosity agent is 2%-6%, 2.5%-6%, 3%-6%, 4%-6%, 3.5%-6%, and 1.5%-6%.

In the above-mentioned technical solution provided by the present invention, the viscosity agent in the polishing composition is selected from at least one of alcohols, silicic acid, silicate, alginic acid, alginate, polyvinylpyrrolidone, polyvinylpyrrolidone-modified organic, ethyl cellulose, methyl cellulose, hydroxyethyl cellulose, hydroxypropylmethyl cellulose, carboxymethyl cellulose, polyacrylic acid, sodium polymethacrylate, polycarboxylic acid sodium salts, carboxylic acid sodium salt copolymers, carboxylic acid sulfonic acid copolymers, guar gum, sodium starch phosphate, sodium polyacrylate, polyoxyethylenes, carbomer, xanthic acid, arabic gum, carrageenan, agar, gelatin, acacia gum, and derivatives thereof. For example, in some embodiments of the present invention, the viscosity agent in the polishing composition is silicic acid, in other examples, the viscosity agent is hydroxypropylmethyl cellulose, and in still other examples, the viscosity agent is a combination of an acacia gum derivative and an alcohol.

In the above-mentioned technical solution of the present invention, the viscosity agent in the polishing composition includes at least one of the A components and at least one of the B components; wherein

the A component is selected from at least one of alcohols, ethyl cellulose, methyl cellulose, hydroxyethyl cellulose, hydroxypropylmethyl cellulose, carboxymethyl cellulose, polyvinylpyrrolidone, polyacrylic acid, polymethacrylate, polycarboxylic acid sodium salts, carboxylic acid sodium salt copolymers, carboxylic acid sulfonic acid copolymers, and derivatives thereof;

the B component is selected from a mixture of at least one of silicic acid, silicate, alginic acid, alginate, polyvinylpyrrolidone-modified organic, guar gum, sodium starch phosphate, sodium polyacrylate, polyoxyethylenes, carbomer, xanthic acid, arabic gum, carrageenan, agar, gelatin, acacia gum, and derivatives thereof.

In some embodiments of the present invention, the viscosity agent is ethyl cellulose and guar gum, and in other examples, the viscosity agent is sodium silicate and sodium polymethacrylate.

In the above-mentioned technical solution provided by the present invention, a mass ratio of the A component to the B component of the viscosity agent in the polishing composition is 0.5: 1-15: 1. Further, the mass ratio of the A component to the B component of the viscosity agent is 0.5: 1-10: 1. Further, the mass ratio of the A component to the B component of the viscosity agent is 1.5: 1-10: 1.

In the above-mentioned technical solution provided by the present invention, the molecular weight Mn of the alcohol in the viscosity agent is ≤ 800 g/mol. Further, the molecular weight of the viscosity agent is ≤ 720 g/mol. Further, the alcohols and derivatives thereof in the present invention refer to alcohols which are liquid at normal temperature, and may be straight-chain alcohols or branched-chain alcohols. The position of the hydroxyl group is also not fixed, such as butanediol, which may be arranged in a straight chain of four methyl groups, methylene groups and/or methylidyne groups, or the carbon chain contains quaternary carbon atoms. In addition, the positions of the two hydroxyl groups may also be arbitrarily transformed on the four carbon atoms, and so on.

In the above-mentioned technical solution provided by the present invention, the alcohols and derivatives thereof are at least one of a monohydric alcohol monomer and a polyhydric alcohol monomer having no more than 6 carbon atoms, and a polymer formed by the monohydric alcohol monomer and the polyhydric alcohol monomer having no more than 6 carbon atoms.

It should be noted that not more than in the present invention means either less than or equal to, and "monohydric alcohol and polyhydric alcohol having no more than 6 carbon atoms" described in the present invention refers to a monohydric alcohol or polyhydric alcohol having less than or equal to 6 carbon atoms.

In the above-mentioned technical solution provided by the present invention, the alcohol and derivatives thereof are at least one of ethylene glycol, propylene glycol, glycerol, butanediol, polyethylene glycol, butanol, cyclohexanol, polypropylene glycol, and polybutylene glycol.

In the above-mentioned polishing method provided by the present invention, the polishing composition further includes a complexing agent, and the complexing agent has a content of 0.5-2%.

In the above-mentioned technical solution provided by the present invention, the complexing agent is selected from at least one of hydroxycarboxylic acid, aminocarboxylic acid, hydroxyaminocarboxylic acid, hydroxyphosphonic acid, dicarboxylic acid, and salts thereof.

In the above-mentioned technical solution provided by the present invention, the complexing agent is selected from at least one of oxalic acid, amino trimethylene phosphonic acid, hydroxyethylidene diphosphonic acid, ethylenediamine tetramethylene phosphonic acid, amino trimethylene phosphonic acid, diethylene triamine pentacarboxylic acid, ethylenediamine tetramethylene phosphonic acid, ethylenediamine tetraacetic acid, diethylene triamine pentamethylene phosphonic acid, amino trimethylene phosphonic acid, hydroxyethylidene diphosphonic acid, 2-hydroxy phosphonoacetic acid, dihexene triamine pentamethylene phosphonic acid, 2-phosphono-1,2,4-butane tricarboxylic acid, heptanoic acid, 2-phosphonobutane-1,2,4-tricarboxylic acid, itaconic acid, succinic acid, tartaric acid, maleic acid, glycolic acid, malonic acid, oxalic acid, malic acid, gluconic acid, alanine, glycine, lactic acid, diethylenetriamine pentaacetic acid, triethylenediamine, propylenediamine tetraacetic acid, hydroxyethyl ethylenediamine, hydroxyethyl ethylenediamine triacetic acid, pyrophosphoric acid, 2-aminoethylphosphonic acid, 1-hydroxyethylidene 1,1-diphosphonic acid, amino trimethylene phosphonic acid, ethylenediamine tetramethylene phosphonic acid, diethylenetriamine pentamethylene phosphonic acid, ethane-1,1-diphosphonic acid, ethane-1,1,2-triphosphonic acid, methane hydroxyphosphonic acid, 1-phosphonobutane-2,3,4-tricarboxylic acid and salts thereof, dihydroxy glycine, disodium EDTA, succinic acid, polyvinyl alcohol, triethanolamine, and salts thereof.

In the above-mentioned polishing method provided by the present invention, the polishing composition further includes a surfactant, and the surfactant has a content of 0.05-1%.

In the above-mentioned technical solution provided by the present invention, the surfactant is selected from at least one of higher fatty acid salts, alkyl sulfonate, alkyl ether carboxylate surfactants, silicone surfactants, alkane sulfate, alkane sulfonate, substituted amine salts, betaine, polyethylene oxide, polyvinyl alcohol, polyvinyl acetate, polyacrylic acid, polyvinyl pyrrolidone, lecithin, amino acid derivatives, alkylphenol polyoxyethylene ether, fatty alcohol polyoxyethylene ether sodium sulfate, isooctanol polyoxyethylene ether, alkyl glucoside, polyoxyethylene ether, polyethyleneimine, alkyl trimethyl quaternary ammonium salts, alkyl dimethyl benzyl ammonium chloride salts, or pyridinium salts.

In the above-mentioned technical solution provided by the present invention, the surfactant is selected from at least one of sodium dodecyl sulfate, sodium dodecyl sulfonate, OP-10, linear alkylbenzene sulfonate (LAS), fatty alcohol polyoxyethylene ether sulfate (AES), fatty alcohol polyoxyethylene ether ammonium sulfate (AESA), sodium lauryl sulfate (SDS), lauroyl glutamic acid, nonylphenol polyoxyethylene ether (TX-10), glycerol monostearate, lignosulfonate, heavy alkylbenzene sulfonate, alkyl sulfonate, diffusant NNO, diffusant MF, alkyl polyether (PO-EO copolymer), fatty alcohol polyoxyethylene ether (AEO-3), betaine, alkyl trimethyl quaternary ammonium salts, lecithin, amino acid derivatives, alkylphenol polyoxyethylene ether, fatty alcohol polyoxyethylene ether sulfate, isooctanol polyoxyethylene ether, alkyl glucoside, polyoxyethylene ether, alkyl dimethyl benzyl ammonium chloride salts, or pyridinium salts.

In the above-mentioned polishing method provided by the present invention, the polishing composition further includes a surface protecting agent, and the surface protecting agent has a content of 0.05-1%.

In the above-mentioned technical solution provided by the present invention, the surface protecting agent is selected from at least one of tannic acid, phytic acid, stearic acid, palmitic acid, and salts thereof. That is, the surface protecting agent may be at least one of tannic acid, phytic acid, stearic acid, palmitic acid, tannate, phytate, stearate, palmitate, or a mixture of at least one of tannic acid, phytic acid, stearic acid, palmitic acid, and at least one of tannate, phytate, stearate, and palmitate. Salts include, but are not limited to, sodium, potassium, ammonium, calcium, or magnesium salts.

It should be noted that a component in the polishing composition of the present invention is selected from at least one of a, b, c, d, and derivatives thereof, or a component in the polishing composition of the present invention is selected from at least one of a, b, c, d, and salts thereof, which refers to that a component in the polishing composition is selected from at least one of a, a derivative, b, b derivative, c, c derivative, d, and d derivative, or a component in the polishing composition is selected from at least one of a, a salt, b, b salt, c, c salt, d, and d salt. For example, "the complexing agent is selected from at least one of hydroxycarboxylic acid, aminocarboxylic acid, hydroxyaminocarboxylic acid, hydroxyphosphonic acid, dicarboxylic acid, and salts thereof" refers to that the complexing agent is selected from at least one of hydroxycarboxylic acid, aminocarboxylic acid, hydroxyaminocarboxylic acid, hydroxyphosphonic acid, dicarboxylic acid, a hydroxycarboxylic acid salt, an aminocarboxylic acid salt, a hydroxyaminocarboxylic acid salt, a hydroxyphosphonic acid salt, and a dicarboxylic acid salt.

In the present invention, a salt refers to a sodium, potassium, ammonium, calcium, or magnesium salt formed by the corresponding acids and the corresponding metal ions. For example, alginates include sodium alginate, potassium alginate, ammonium alginate, calcium alginate, and magnesium alginate. Silicates include aluminum silicate, iron silicate, calcium silicate, magnesium silicate, potassium silicate, sodium silicate, and so on.

In the present invention, "/" means "or". For example, "interventional material/device" means "interventional material or interventional device".

Beneficial effects of the present invention are as follows:
1) In the present invention, controlling the removal amount and uniformity in the polishing process ensures that cracks and the like deep inside the pre-polishing member can be sufficiently and completely removed, thereby preventing problems such as the scrapping of the polishing member due to the failure of certain property indicators in use caused by internal cracks. In addition, it is ensured that the thicknesses, etc., of various parts of the polishing member are kept consistent, thereby ensuring that the polishing member has good mechanical properties. This prevents uneven mechanical properties of various parts of the polishing member due to some parts being too thin and others too thick, which may lead to safety accidents.
2) The medical device polishing member has a smooth surface without burr, round edges, and good mechanical properties. In addition, the surface of the polishing member is smooth without burr, and the edges are round and smooth. The polishing member has relatively high safety during implantation into the human body and small damage to the human body.
3) In the present invention, the polishing member is directly formed in one step, and the polishing process is not divided into several steps to complete and is simple in operation. Meanwhile, a thin protective film may be formed on the surface of the polished polishing member while polishing the polishing member to prevent oxidation reaction and the like on the surface of the polishing member during transportation or storage, thereby increasing the stability of surface properties, appearance, and the like.
4) In the present invention, the polishing process is simple and easy to be industrialized, the polishing conditions are mild, the polishing time is suitable and controllable, the reproducibility is good, and the precision of the polished stent is controlled. A plurality of identical stents are polished under the same conditions, and the RSD of the mass/weight of the plurality of polished stents is within 2%, even within 0.8%.

In the present invention, the value of the interval range is not limited to the interval range provided, but should be the value of a new interval composed of any two values in the interval or any specific value in the interval. For example, "the content of the surfactant is 0.05-0.1%" in the present invention refers to that the value of the content of the surfactant is not limited to the interval range of 0.05-1% and may be a new interval composed of any two values in the infinite number of values between 0.05-1%, such as may be 0.05-0.5 or 0.1-0.8. In addition, when there are a plurality of numerical combinations, each parameter may have any value within its value range, and the values of the plurality of parameters may be arbitrarily matched. If the pH value of the polishing solution is ≤ 1, the viscosity of the polishing solution is 1-4 mPa s. The pH value of the polishing solution may be any value within a range of values not greater than 1, and the viscosity of the polishing solution may have any value within a range of 1-4 mPa s. The two parameters may also be arbitrarily matched. For example, the pH value is 0.1, and the viscosity of the polishing solution is 3.5 mPa s, and so on.

It should be understood that the terms used herein are for the purpose of describing particular exemplary embodiments only and are not intended to form a limitation. As used herein, the singular forms "a", "an", and "the" are intended to include the plural forms as well, unless the context clearly indicates otherwise. The terms "comprising", "including", "containing", and "having" are inclusive, and therefore specify the presence of stated features, steps, operations, elements, and/or components, but do not preclude the presence or addition of one or more other features, steps, operations, elements, components, and/or combinations thereof. The method steps, processes, and operations described herein are not to be construed as necessarily requiring that they be performed in the particular order described or illustrated, unless the order of performance is explicitly stated. It should also be understood that additional or alternative steps may be used.

### Brief Description of the Drawings

Various other advantages and benefits will become apparent to a person skilled in the art upon reading the following detailed description of the preferred embodiments. The drawings are only for the purpose of illustrating the preferred embodiments and are not to be construed as limiting the present invention. Moreover, throughout the accompanying drawings, the same components are indicated by the same reference numerals.

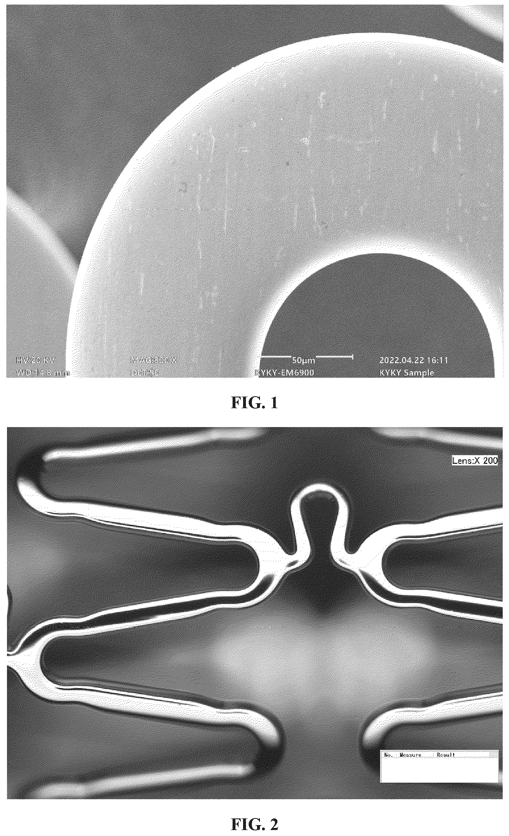

FIG. 1 is a characterization diagram of a stent of example 1 at a magnification of 500 times under an SEM;
FIG. 2 is a characterization diagram of a stent of example 1 at a magnification of 200 times under an optical microscope; and

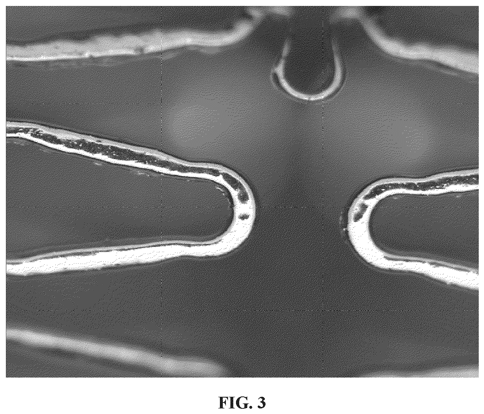

FIG. 3 is a characterization diagram of a stent of comparative example 1 at a magnification of 200 times under an optical microscope.

### Detailed Description of the Invention

Hereinafter, exemplary embodiments of the present invention will be described in more detail with reference to the accompanying drawings. While exemplary embodiments of the present invention have been illustrated in the accompanying drawings, it is to be understood that the present invention may be embodied in various forms and should not be construed as limited to the embodiments set forth herein. On the contrary, these embodiments are provided to enable a thorough understanding of the present invention and to convey the scope of the present invention completely to a person skilled in the art.

### Test methods

### 1. Roughness

In the present invention, the roughness of a surface of a polishing member is measured using a Q-SIX cardiovascular stent detector manufactured by SENSOFAR under an interference lens at a magnification of 400 times and a scanning height of 50 µm.

### 2. Viscosity

A polishing solution is poured into a Zahn cup CUP1#, and the outflow time t is recorded. The viscosity of the solution is calculated according to the formula: dynamic viscosity = 1.1(t-29) * ρ (ρ refers to the solution density).

### 3. Measurement of width and thickness of stent

A stent is placed under an optical microscope, and the size of the stent is observed and measured under the optical microscope at a magnification of 100-200 times.

### 4. Scratch depth

The stent is sealed and then polished using a polishing machine until a cross section of the stent is exposed. Finally, the polished cross section is placed under a metallographic microscope to measure the scratch depth.

### 5. Radial strength

In the property test of the lumen stent, the radial strength of the lumen stent can be tested by applying radial pressure uniformly to the stent using a compression module, causing the stent to compress and undergo uniform deformation. The radial strength of the stent is defined as the magnitude of the radial pressure applied when 10% deformation occurs in the radial direction (outer diameter) of the stent.

### 6. Over-expansion plasticity

A balloon catheter with the corresponding length and suitable outer diameter is selected for the stent. The balloon catheter is pressurized and expanded from 6-8 atm, and after holding the pressure for 30 s, the whole stent is traversed under a 200× three-dimensional measuring microscope to record whether there are cracks/broken rods. If there are no cracks/broken rods, the operation of holding the pressure for 30 s after pressurizing for 2 atm for observation and recording is repeated until cracks/broken rods are found or the expanded outer diameter of the stent reaches the acceptance criteria.

### 7. Appearance

The brightness and roughness of the outer and inner walls of the whole stent are fully examined through the three-dimensional measuring microscope at an appropriate magnification (such as 50-200 times).

### Example 1

A 30018 stent was cut, placed in an ultrasonic wave for cleaning for 2 min, and then placed in a polishing solution with a viscosity of 4.0 mPa s, which was prepared by mixing 100 ml of sulfuric acid, 3 g of EDTA, 10 ml of glycerol, 5 g of sodium nitrate, 5 g of sodium silicate, 0.1 ml of phytic acid, 0.05 g of sodium dodecyl sulfate, and 50 ml of water. A flow rate of the polishing solution was controlled to be 1.0 m/s, and the stent was polished at 20°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 1 µm, the single-side removal depth of a stent rod was 15 µm, and the uniformity of stent polishing was 90%. A mass loss rate of the stent was 45%, and the roughness Sa of the surface of the stent measured under a sensofar 3D optical profiler was 15 nm. The stent had a radial strength of 120 kPa and was expanded using a balloon to a diameter of 4.6 mm without cracks and fracture. Five stents were consecutively and repeatedly polished, and the RSD of the mass of the polished stents was < 1%.

### Example 2

A 30018 stent was cut, placed in an ultrasonic wave for cleaning for 2 min, and then placed in a polishing solution with a viscosity of 2.0 mPa s, which was prepared by mixing 100 ml of hydrochloric acid, 5 g of tartaric acid, 10 ml of ethylene glycol, 10 ml of perchloric acid, 5 g of sodium alginate, 0.1 g of sodium phytate, 0.05 g of OP-10, and 50 ml of water. A flow rate of the polishing solution was controlled to be 0.8 m/s, and the stent was polished at 25°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 0.5 µm, the single-side removal depth of a stent rod was 15 µm, and the uniformity of stent polishing was 95%. A mass loss rate of the stent was 42%, and the roughness Sa of the surface of the stent measured under a sensofar 3D optical profiler was 10 nm. The stent had a radial strength of 115 kPa and was expanded using a balloon to a diameter of 4.6 mm without cracks and fracture. Five stents were consecutively and repeatedly polished, and the RSD of the mass of the polished stents was < 1.5%. Five stents were consecutively and repeatedly polished, and the RSD of the mass of the polished stents was < 1.5%.

### Example 3

A 30018 stent was cut, placed in an ultrasonic wave for cleaning for 5 min, and then placed in a polishing solution with a viscosity of 3.0 mPa s, which was prepared by mixing 100 ml of phosphoric acid, 5 g of disodium EDTA, 10 ml of butanediol, 10 ml of nitric acid, 5 g of sodium silicate, 0.1 ml of tannic acid, 0.05 g of polyethylene glycol, and 50 ml of water. A flow rate of the polishing solution was controlled to be 0.5 m/s, and the stent was polished at 30°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 1.5 µm, the single-side removal depth of a stent rod was 15 µm, and the uniformity of stent polishing was 92%. A mass loss rate of the stent was 45%, and the roughness Sa of the surface of the stent measured under a sensofar 3D optical profiler was 20 nm. The stent had a radial strength of 125 kPa and was expanded using a balloon to a diameter of 4.6 mm without cracks and fracture. Five stents were consecutively and repeatedly polished, and the RSD of the mass of the polished stents was < 2%.

### Example 4

A 30018 stent was cut, placed in an ultrasonic wave for cleaning for 2 min, and then placed in a polishing solution with a viscosity of 1.5 mPa s, which was prepared by mixing 100 ml of hydrochloric acid, 3 g of succinic acid, 10 ml of propylene glycol, 10 ml of hydrogen peroxide, 5 g of sodium silicate, 0.1 g of stearic acid, 0.05 g of sodium dodecyl sulfonate, and 50 ml of water. A flow rate of the polishing solution was controlled to be 0.5 m/s, and the stent was polished at 30°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 2 µm, the single-side removal depth of a stent rod was 15 µm, and the uniformity of stent polishing was 92%. A mass loss rate of the stent was 44%, and the roughness Sa of the surface of the stent measured under a sensofar 3D optical profiler was 20 nm. The stent had a radial strength of 120 kPa and was expanded using a balloon to a diameter of 4.6 mm without cracks and fracture. There was no obvious change after the stent was stored for 4 weeks. Five stents were consecutively and repeatedly polished, and the RSD of the mass of the polished stents was < 2.5%.

### Example 5

A 30018 stent was cut, ultrasonically cleaned for 2 min with 12.5% hydrochloric acid, and then placed in a solution with a viscosity of 1.0 mPa s, which contained 100 ml of hydrofluoric acid, 3 g of diethylene triamine pentamethylene phosphonic acid, 10 ml of cyclohexanol, 10 ml of potassium permanganate, 5 g of sodium alginate, 0.1 g of palmitic acid, and 50 ml of pure water. The solution was placed in a low-temperature magnetic groove at a temperature of 30°C. The polishing solution flowed at a rate of 0.8.0 m/s, and the stent was placed in the polishing solution for polishing for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 0.5 µm, the single-side removal depth of a stent rod was 15 µm, and the uniformity of stent polishing was 92%. A mass loss rate of the stent was 46%, and the roughness Sa of the surface of the stent measured under a sensofar 3D optical profiler was 20 nm. The stent had a radial strength of 115 kPa and was expanded using a balloon to a diameter of 4.6 mm without cracks and fracture. Five stents were consecutively and repeatedly polished, and the RSD of the mass of the polished stents was < 3%.

### Example 6

A 30018 stent was cut, ultrasonically cleaned for 2 min with 12.5% hydrochloric acid, and then placed in a solution with a viscosity of 2.5 mPa s, which contained 100 ml of perchloric acid, 5 g of sodium gluconate, 10 ml of butanol, 5 g of potassium nitrate, 5 g of sodium alginate, 0.1 g of sodium stearate, 0.05 g of betaine, 0.05 g of sodium lauryl sulfate, and 50 ml of pure water. The solution was placed in a low-temperature magnetic groove at a temperature of 25°C. The polishing solution flowed at a rate of 0.6.0 m/s, and the stent was placed in the polishing solution for polishing for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 0.3 µm, the single-side removal depth of a stent rod was 15 µm, and the uniformity of stent polishing was 95%. A mass loss rate of the stent was 43%, and the roughness Sa of the surface of the stent measured under a sensofar 3D optical profiler was 12 nm. The stent had a radial strength of 118 kPa and was expanded using a balloon to a diameter of 4.6 mm without cracks and fracture. Five stents were consecutively and repeatedly polished, and the RSD of the mass of the polished stents was < 3%.

### Example 7

A 30018 stent was cut, placed in an ultrasonic wave for cleaning for 2 min, and then placed in a polishing solution with a viscosity of 2.5 mPa s, which was prepared by mixing 100 ml of sulfuric acid, 5 g of disodium EDTA, 10 ml of ethylene glycol, 5 g of sodium nitrate, 0.1 ml of phytic acid, and 50 ml of water. A flow rate of the polishing solution was controlled to be 0.5 m/s, and the stent was polished at 25°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2.5%, the maximum depth of the scratch was 2 µm, the single-side removal depth of a stent rod was 15 µm, and the uniformity of stent polishing was 85%. A mass loss rate of the stent was 44%, and the roughness Sa of the surface of the stent measured under a sensofar 3D optical profiler was 18 nm. The stent had a radial strength of 115 kPa and was expanded using a balloon to a diameter of 4.5 mm with a fracture. Five stents were consecutively and repeatedly polished, and the RSD of the mass of the polished stents was < 3%.

### Example 8

A 30018 stent was cut, placed in an ultrasonic wave for cleaning for 2 min, and then placed in a polishing solution with a viscosity of 3.5 mPa s, which was prepared by mixing 100 ml of hydrochloric acid, 3 g of succinic acid, 10 ml of glycerol, 10 ml of nitric acid, 5 g of sodium alginate, and 50 ml of water. A flow rate of the polishing solution was controlled to be 0.6 m/s, and the stent was polished at 30°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 1.2 µm, the single-side removal depth of a stent rod was 1.5 µm, and the uniformity of stent polishing was 88%. A mass loss rate of the stent was 48%, and the roughness Sa of the surface of the stent measured under a sensofar 3D optical profiler was 25 nm. The stent had a radial strength of 120 kPa and was expanded using a balloon to a diameter of 4.6 mm without cracks and fracture. The stent had yellow spots on the surface after 7 days of storage. Five stents were consecutively and repeatedly polished, and the RSD of the mass of the polished stents was < 2%.

### Example 9

A 30018 stent was cut, placed in an ultrasonic wave for cleaning for 2 min, and then placed in a polishing solution with a viscosity of 3.5 mPa s, which was prepared by mixing 100 ml of phosphoric acid, 5 g of malic acid, 10 ml of butanediol, 10 ml of perchloric acid, and 50 ml of water. A flow rate of the polishing solution was controlled to be 0.8 m/s, and the stent was polished at 35°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 3%, the maximum depth of the scratch was 2.5 µm, the single-side removal depth of a stent rod was 15 µm, and the uniformity of stent polishing was 82%. A mass loss rate of the stent was 48%, and the roughness Sa of the surface of the stent measured under a sensofar 3D optical profiler was 30 nm. The stent had a radial strength of 128 kPa and was expanded using a balloon to a diameter of 4.0 mm with a fracture. Five stents were consecutively and repeatedly polished, and the RSD of the mass of the polished stents was < 1.5%.

### Comparative example 1

A 30018 stent was cut, placed in an ultrasonic wave for cleaning for 2 min, and then placed in a polishing solution, which was prepared by mixing 140 ml of glacial acetic acid and 60 ml of perchloric acid. The stent was polished using a direct current power supply at a constant current of 0.1 A for 20 s. The stent was removed, cleaned, and dried. The dried stent was observed under an optical microscope with a magnification of 200 times to find that the surface was smooth and bright. However, the stent was locally yellow and had small sizes at two ends and a large size in the middle. The dried stent was observed under an SEM with a magnification of 500 times to find that edges of the stent were round, the percentage of a surface scratch area was 5%, the maximum depth of the scratch was 6 µm, the single-side removal depth of an inner wall of a stent rod was 5 µm, and the uniformity of stent polishing was 50%. A mass loss rate of the stent was 50%, and the roughness Sa of the surface of the stent measured under a sensofar 3D optical profiler was 200 nm. The stent had a radial strength of 95 kPa and was expanded using a balloon to a diameter of 3.5mm with a fracture. Five stents were consecutively and repeatedly polished, and the RSD of the mass of the polished stents was < 3%.

### Comparative example 2

A 30018 stent was cut and placed in an ultrasonic wave for cleaning for 2 min. A polishing solution prepared by mixing 60 ml of phosphoric acid, 30 ml of sulfuric acid, and 10 ml of nitric acid was heated to boiling. The stent was placed in the heated polishing solution for polishing for 10 s, cleaned, and dried. The dried stent was observed under an optical microscope with a magnification of 200 times to find that the surface was rough, pitted, and yellow. The dried stent was observed under an SEM with a magnification of 500 times to find that edges of the stent were round, the percentage of a surface scratch area was 8%, the maximum depth of the scratch was 0.5 µm, the single-side removal depth of an inner wall of a stent rod was 16 µm, and the uniformity of stent polishing was 90%. A mass loss rate of the stent was 65%, and the roughness Sa of the surface of the stent measured under a sensofar 3D optical profiler was 250 nm. The stent had a radial strength of 70 kPa and was expanded using a balloon to a diameter of 4.0 mm with a fracture. Five stents were consecutively and repeatedly polished, and the RSD of the mass of the polished stents was > 10%.

It can be seen from the comparative examples that in comparative example 1, after electrochemical polishing, the sizes of various parts of the stent are uneven, the surface is yellow and relatively rough, the percentage of the surface scratch area is relatively high, the scratch has a large depth, and both the radial support force and the over-expansion capability of the stent are relatively low. In comparative example 2, after the high-temperature chemical polishing, the surface of the stent is yellow and rough, and the percentage of the surface scratch area of the polishing member is also relatively high. Meanwhile, the polishing time of this method is very short, only 5 s, and the polishing precision of the product is not high. After repeated polishing for many times, the RSD of product size and quality among multiple polished products is relatively large, and the polishing stability is poor.

The above are only preferred examples of the present invention and are not intended to limit the present invention. For example, the examples of the present invention are all illustrated by using a stent as an example, which does not mean that the solution of the present application is only suitable for the polishing of the stent. The solution of the present application may also be suitable for the polishing of precision parts of other medical devices and even other non-medical devices with a small mass. Although the present invention has been described in detail with reference to the foregoing examples, a person skilled in the art will be able to make modifications to the technical solutions described in the foregoing examples or make equivalents to some of the technical features thereof. Any modifications, equivalents, improvements, etc. made within the spirit and principles of the present invention should be included within the scope of the present invention.

## Claims

1. A medical device polishing member, wherein the medical device polishing member has a mass loss rate of 20-60% in a polishing process.

2. The medical device polishing member according to claim 1, wherein the medical device polishing member has a polishing uniformity of more than 85% in the polishing process.

3. The medical device polishing member according to claim 1, wherein a single-side removal depth after polishing an inner wall, outer wall, and side wall of the medical device polishing member is ≥ 1.5 µm.

4. The medical device polishing member according to claim 1, wherein the polishing member has a smooth bright surface after being magnified 100 times under an optical electron microscope.

5. The medical device polishing member according to claim 1, wherein the polishing member has a slight scratch on a surface after being magnified 500 times under a scanning electron microscope; a depth h of the scratch on the surface of the polishing member is ≤ 2.5 µm.

6. The medical device polishing member according to claim 1, wherein the roughness Sa of a surface of the medical device polishing member is ≤ 50 nm.

7. The medical device polishing member according to claim 1, wherein edges of the medical device polishing member are round and smooth; the edges of the medical device polishing member have circular arc shapes.

8. The medical device polishing member according to claim 1, wherein a protective film is attached to a surface of the medical device polishing member.

9. The medical device polishing member according to claim 1, wherein a mass-to-volume ratio of the medical device polishing member is 0.001-10 g/cm³; the mass-to-volume ratio of the medical device polishing member is 0.001-5 g/cm³; the mass-to-volume ratio of the medical device polishing member is 0.001-0.4 g/cm³.

10. The medical device polishing member according to claim 1, wherein the medical device polishing member is an interventional device or an implant device; the medical device polishing member is made of one of degradable metals or metal alloys; the medical device comprises any one of a vascular stent, a heart valve, a non-vascular endoluminal stent, an occluder, an orthopedic implant, a dental implant, a respiratory implant, a gynecological implant, an andrological implant, a suture, or a bolt.

11. A polishing method for preparing the medical device polishing member according to any one of claims 1-10, comprising the steps of:
a) performing polishing pretreatment on a device base;
b) placing a pretreated device base into a polishing solution containing an acidic polishing composition for polishing; and
c) cleaning and drying a polished device base to obtain a device polishing member.

12. The polishing method according to claim 11, wherein a pH value of the polishing solution in step b) is ≤ 1; the viscosity of the polishing solution in step b) is 1-4 mPa s.

13. The polishing method according to claim 11, wherein a temperature of the polishing in step b) is 20-35°C; a time of the polishing in step b) is 1-20 min; the polishing solution in step b) is kept flowing at a rate of 0.1-1.5 m/s in the polishing process.

14. The polishing method according to claim 11, wherein the polishing pretreatment refers to ultrasonic treatment in an acidic solution for 2-10 min.

15. The polishing method according to claim 11, wherein the polishing composition comprises an acid, an oxidizing agent, and a viscosity agent; based on a total mass fraction of the polishing composition being 100%, the composition of components is as follows:
| Components | Mass percentage content (.wt%) |
|---|---|
| Acid | 40-60% |
| Oxidizing agent | 5-10% |
| Viscosity agent | 1.5-7% |
| Water | Balance |

16. The polishing method according to claim 15, wherein the polishing composition further comprises a complexing agent, and the complexing agent has a content of 0.5-2%.

17. The polishing method according to claim 15, wherein the polishing composition further comprises a surfactant, and the surfactant has a content of 0.05-1%.

18. The polishing method according to claim 15, wherein the polishing composition further comprises a surface protecting agent, and the surface protecting agent has a content of 0.02-1%.

19. The polishing method according to claim 15, wherein the viscosity agent comprises a main viscosity agent and an auxiliary viscosity agent, wherein a mass ratio of the main viscosity agent and the auxiliary viscosity agent is 0.5: 1-15: 1.
